# EUROPÄISCHE PATENTSCHRIFT

(11) **EP 2 151 202 B1**
(45) Veröffentlichungstag und Bekanntmachung des Hinweises auf die Patenterteilung: **16.02.2011**
(21) Anmeldenummer: 08161738.3
(22) Anmeldetag: 04.08.2008
(51) Int. Cl.: A61B 17/16

(54) **Klemmhülse zum Klemmen eines kanülierten Bohrers und eines Führungsdrahtes**
Clamping piece for clamping a cannulated drill and a guide wire
Douille de blocage destinée au blocage d'un foret canulé et d'un fil en acier de guidage

(43) Veröffentlichungstag der Anmeldung: 10.02.2010
(73) Patentinhaber: BrainLAB AG, 85622 Feldkirchen (DE)
(72) Erfinder: Christian, Georg, 81545, München (DE); Uhde, Jörg, 80538, München (DE)
(74) Vertreter: Schwabe - Sandmair - Marx

(56) Entgegenhaltungen:
- US-A1- 2004 122 460
- US-A1- 2006 286 504
- US-B1- 7 207 995

## Beschreibung

Die vorliegende Erfindung betrifft eine Klemmhülse zum Klemmen eines kanülierten Bohrers und eines Führungsdrahtes, insbesondere eines Kirschnerdrahtes. Die Klemmhülse dient also insbesondere medizinischen Zwecken. Die Klemmhülse soll ein gleichzeitiges Klemmen des kanülierten Bohrers und des Führungsdrahtes bewirken, so dass der kanülierte Bohrer relativ zum Führungsdraht fixiert ist.

Bei bekannten kanülierten Bohrern wird der Führungsdraht lose durch den Bohrer geführt. Insbesondere dient bei dem bekannten Verfahren, der Führungsdraht der Führung des Bohrers.

US 7,207,995 B1 offenbart ein kanüliertes medizinisches Instrument zum Einführen eines medizinischen Fixationselements mittels eines Führungsdrahtes. In diesem Instrument kann eine Klemmhülse verwendet werden, um den Führungsdraht zu sichern.

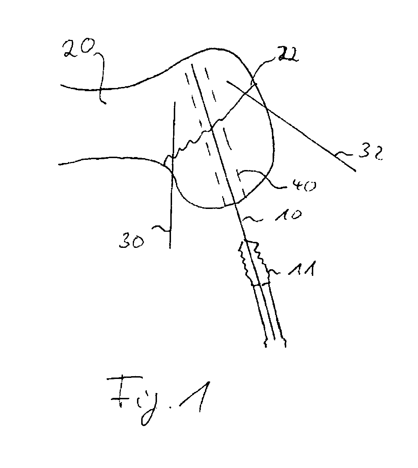

Figur 1 zeigt schematisch eine Fraktur 22 oder Fissur 22 eines Knochens 20. Die Knochenfraktur 22 wird mit zwei Kirschnerdrähten 30 und 32 fixiert. Danach wird beim Stand der Technik ein weiterer Kirschnerdraht 10 eingeführt. Die Lage des eingeführten Kirschnerdrahts 10 wird mittels eines Röntgengeräts oder C-Bogens kontrolliert. Falls sich durch die Kontrolle ergibt, dass die Lage nicht korrekt ist, wird der Kirschnerdraht 10 herausgezogen und erneut eingeschoben, bis sich bei Kontrolle durch das Röntgengerät eine akzeptable Lage des Kirschnerdrahtes 10 ergibt. Eine Fehllage des Kirschnerdrahtes 10 kann sich auch ergeben, wenn der Kirschnerdraht 10 navigiert wird, da der Kirschnerdraht 10 im Knochen 20 gebogen werden kann. Eine Navigation bedeutet hier beispielsweise das Anbringen von Markerelementen oder einer Markeranordnung bestehend aus mehreren Markerelementen an einem Teil des Kirschnerdrahtes oder an einem fest mit dem Kirschnerdraht verbundenem Gegenstand. Dies ist beispielsweise bei einem in einem Bohrfutter eingespannten Draht die dazugehörige Bohrmaschine und eine Markeranordnung an der Zielregion, zum Beispiel dem frakturierten Knochen. Die Lage der Markerelemente wird durch eine Detektionseinrichtung detektiert. Die Markerelemente können aktiv Strahlen oder Wellen (z.B. Infrarotstrahlen) aussenden oder passiv Strahlen oder Wellen (z.B. Infrarotstrahlen) reflektieren. Die ausgesandten oder reflektierten Strahlen oder Wellen werden durch einen Detektor (z.B. Infrarotkamera) detektiert. Aus den Detektionssignalen lässt sich so die Lage der Markerelemente bestimmen. Da die relative Lage zwischen den Markerelementen und dem zu navigierenden Teil (hier: die Spitze des Kirschnerdrahts) bekannt ist, kann die Lage des Kirschnerdrahts 10 bestimmt werden. Hierbei allerdings unter der Voraussetzung, dass der Kirschnerdraht 10 nicht gebogen wird.

Ist man mit der Lage des Kirschnerdrahtes 10 einverstanden, so wird ein kanülierter Bohrer 11 über den Kirschnerdraht geführt, um mittels einer Bohrmaschine eine Bohrung 40, die in Figur 1 gestrichelt gezeichnet ist, am Knochen vorzunehmen. Ist die Bohrung vollendet, so wird der Bohrer entfernt und über den Kirschnerdraht eine Schraube eingefädelt, um die Knochenfraktur mittels der Schraube an der vorgesehenen Position zu fixieren. Der Kirschnerdraht unterstützt die korrekte Positionierung der Schraube beim Einschrauben der Schraube in den gefertigten Bohrkanal.

Aufgabe der Erfindung ist es, zu ermöglichen, beim Bohrvorgang den Führungsdraht zusammen mit dem Bohrer in ein Objekt einzubohren.

Vorstehende Aufgabe wird durch den Gegenstand des Anspruches 1. Die abhängigen Ansprüche sind auf vorteilhafte Ausführungsformen gerichtet.

Vorteilhaft kann die erfindungsgemäße Klemmhülse in das Bohrfutter einer Bohrmaschine eingebracht werden. Sie liegt also zwischen der Innenoberfläche des Bohrfutters und der Außenoberfläche des kanülierten Bohrers und der Außenoberfläche des nicht vom kanülierten Bohrer umhüllten Führungsdrahtes.

Die Klemmhülse umfasst vorzugsweise mindestens eine Bohrer-Kontaktfläche, die dazu dient den kanülierten Bohrer zu fixieren, wenn die Bohrer-Kontaktfläche gegen den kanülierten Bohrer gedrückt wird. Die Bohrer-Kontaktfläche steht vorzugsweise in das Innere der Klemmhülse vor, insbesondere in radialer Richtung. Die Bohrer-Kontaktfläche leitet eine Kraft, die auf die Außenseite der Klemmhülse wirkt, die insbesondere von dem Bohrfutter ausgeübt wird, auf den kanülierten Bohrer weiter, so dass sich insbesondere eine kraftschlüssige Verbindung zwischen dem kanülierten Bohrer und der Klemmhülse ergibt. Alternativ oder zusätzlich können Vertiefungen oder Erhebungen im kanülierten Bohrer und in der Bohrer-Kontaktfläche ausgebildet sein, die ineinander greifen, so dass sich eine formschlüssige Verbindung ergibt.

Der kanülierte Bohrer hat eine Bohrspitze, die sich an einem Ende (erstes Ende) des Bohrers befindet. Zumindest aus dem anderen Ende (zweites Ende) ragt der Führungsdraht hervor. Das zweite Ende des Bohrers umfasst den Bohrerschaft. Aus dem ersten Ende kann insbesondere eine Spitze des Führungsdrahtes hervorragen. Die Bohrer-Kontaktfläche der Klemmhülse kontaktiert vorzugsweise den Bohrerschaft während die Draht-Kontaktfläche den Führungsdraht kontaktiert, der aus dem zweiten Ende des Bohrers hervorragt. Aus dem einen Ende (ersten Ende) der Klemmhülse ragt vorzugsweise ein Teil des Bohrers, insbesondere die Bohrspitze hervor, während aus dem anderen Ende (zweiten Ende) insbesondere der Führungsdraht hervorragt. Die Längsrichtung der Klemmhülse verläuft von dem ersten Ende zu dem zweiten Ende der Klemmhülse. Entlang dieser Längsrichtung liegen vorzugsweise die Bohrer-Kontaktfläche und die Draht-Kontaktfläche hintereinander. Das erste Ende wird hierin auch als erstes Längsende bezeichnet. Das zweite Ende wird hierin auch als zweites Längsende bezeichnet.

Die Draht-Kontaktfläche und/oder die Bohrer-Kontaktfläche stehen vorzugsweise in radialer Richtung nach innen vor. Vorzugsweise steht die mindestens eine Draht-Kontaktfläche weiter nach innen vor als die mindestens eine Bohrer-Kontaktfläche.

Die Draht-Kontaktfläche hat also einen geringeren Abstand zu der in Längsrichtung verlaufenden (virtuellen) Längsachse der Klemmhülse.

Vorzugsweise sind die Bohrer-Kontaktfläche und die Draht-Kontaktfläche so ausgebildet, dass sie einen satten, insbesondere form- und/oder kraftschlüssigen Kontakt mit dem Bohrer und/oder dem Führungsdraht ermöglichen oder gewährleisten, insbesondere wenn eine äußere, nach Innen wirkende Kraft auf die Klemmhülse ausgeübt wird. Ist der Bohrerschaft zylindrisch ausgebildet, so ist die Oberfläche der Bohrer-Kontaktfläche vorzugsweise ebenfalls zylindrisch, also wie ein Zylinderflächenabschnitt ausgebildet. Ist der Führungsdraht zylindrisch ausgebildet, so ist vorzugsweise die Oberfläche der Draht-Kontaktfläche ebenfalls zylindrisch ausgebildet, ist also in der Form eines Zylinderflächenabschnittes, der den Führungsdraht umgibt. Auch kann eine formschlüssige Verbindung zwischen Bohrer und Bohrer-Kontaktfläche und/oder Führungsdraht und Draht-Kontaktfläche ausgebildet werden. Hierzu können beispielsweise Erhöhungen oder Vertiefungen entlang des Umfangs des Bohrers und/oder des Führungsdrahtes ausgebildet sein, in die passende, insbesondere komplementär ausgebildete Fortsätze oder Vertiefungen der Klemmhülse eingreifen.

Vorzugsweise ist die Klemmhülse elastisch gespreizt. Sie weitet sich also insbesondere zum ersten Ende hin, wobei diese Weitung aufgrund der elastischen Ausbildung der Klemmhülse reduziert oder vollständig beseitigt werden kann, insbesondere wenn eine äußere Kraft wirkt. Insbesondere zeigt sich die Weitung darin, dass die Draht-Kontaktfläche und/oder die Bohrer-Kontaktfläche gegenüber der Längsachse der Klemmhülse geneigt ist. Die Neigung ist vorzugsweise so, dass Oberflächenpunkte auf der Oberfläche der Draht-Kontaktfläche und/oder der Bohrer-Kontaktfläche sich in Längsrichtung der Klemmhülse in Richtung auf das erste Ende der Klemmhülse (das der Bohrerspitze näher ist als das zweite Ende der Klemmhülse) zunehmend von der Längsachse der Klemmhülse entfernen. Die vorgenannte Spreizung und/oder Neigung und die damit verbundene zunehmende Entfernung von der Längsachse gilt vorzugsweise in dem Zustand der Klemmhülse, in dem keine Kraft auf sie wirkt. Die Klemmhülse ist vorzugsweise so ausgebildet, dass durch Einwirken einer äußeren Kraft der gespreizte Zustand aufgrund der Elastizität und/oder Verformbarkeit der Klemmhülse so korrigierbar ist, dass die Oberflächen der Bohrer-Kontaktfläche und/oder der Draht-Kontaktfläche parallel zu den Oberflächen des Bohrers und/oder des Führungsdrahtes verlaufen. Insbesondere soll sich dann ein paralleler Verlauf ergeben, wenn Bohrer-Kontaktfläche und/oder Draht-Kontaktfläche jeweilig mit dem Bohrer und/oder Draht vollständig in Kontakt sind.

Vorzugsweise ist die Klemmhülse in ihrer Längsrichtung in Sektoren unterteilt. Diese Sektoren entfernen sich insbesondere (im Mittel) zunehmend von der Längsachse der Klemmhülse, und zwar ausgehend vom zweiten Ende der Klemmhülse zum ersten Ende hin. Vorzugsweise umfasst mindestens ein Sektor mindestens eine Bohrer-Kontaktfläche und mindestens eine Draht-Kontaktfläche. Vorzugsweise umfasst jeder Sektor mindestens eine Bohrer-Kontaktfläche und mindestens eine Draht-Kontaktfläche. Vorzugsweise sind die Sektoren (z.B. stoffschlüssig und/oder einstückig) an einem Ende, vorzugsweise dem zweiten Ende der Klemmhülse miteinander verbunden. Die Sektoren sind über einem weiten Bereich der Längserstreckung der Klemmhülse voneinander beabstandet, insbesondere durch einen Spalt getrennt, falls keine Kraft auf die Klemmhülse von außen nach innen wirkt. Dieser Längsbereich der Klemmhülse, für den eine Beabstandung zwischen den Sektoren gegeben ist, erstreckt sich vorzugsweise über mehr als 50 % der Länge der Klemmhülse vorzugsweise über mehr als 80 % der Länge der Klemmhülse.Vorzugsweise ist eine Beabstandung am ersten Ende der Klemmhülse gegeben und erstreckt sich von dort aus in Richtung des zweiten Endes. Die Beabstandung kann insbesondere als ein vom ersten zum zweiten Ende spitz zulaufender Spalt ausgebildet sein.

Vorzugsweise ist die Beabstandungen der Sektoren so ausgebildet, dass bei einer Krafteinwirkung von außen nach innen sich die Sektoren aufeinander zu bewegen, bis sie sich gegenseitig näher kommen oder berühren. Vorzugsweise ist die Bohr-Kontaktfläche und/oder die Draht-Kontaktfläche so ausgebildet, dass sich bei Berührung der Sektoren in Längsrichtung der Klemmhülse gesehen eine parallele Oberfläche der Draht-Kontaktfläche und/oder der Bohrer-Kontaktfläche einstellt, die parallel zur Längsachse der Klemmhülse ist.

Die Draht-Kontaktfläche ist Bestandteil eines Draht-Kontakt-Abschnittes der Klemmhülse, der sich von der Draht-Kontaktfläche (radial) nach außen zur Außenfläche der Klemmhülse erstreckt. Die Bohrer-Kontaktfläche ist Bestandteil eines BohrerKontakt-Abschnittes der Klemmhülse, der sich von der Bohrer-Kontaktfläche (radial) nach außen zu der Außenfläche der Klemmhülse erstreckt. Der Drahtkontakt-Abschnitt ist mit einem ersten Verbindungsabschnitt mit einem Ende der Klemmhülse, insbesondere dem zweiten Ende verbunden. Der Draht-Kontakt-Abschnitt ist weiter vorzugsweise mit einem zweiten Verbindungsabschnitt mit dem Bohrerkontakt-Abschnitt verbunden. Vorzugsweise ist der erste Verbindungsabschnitt elastischer ausgebildet als der Drahtkontakt-Abschnitt und/oder der Bohrerkontakt-Abschnitt. Vorzugsweise ist der zweite Verbindungsabschnitt elastischer ausgebildet als der Drahtkontakt-Abschnitt und/oder der Bohrerkontakt-Abschnitt. Insbesondere kann der erste Verbindungsabschnitt eine andere Elastizität aufweisen als der zweite Verbindungsabschnitt. Beispielsweise kann der erste Verbindungsabschnitt elastischer ausgebildet sein als der zweite Verbindungsabschnitt.

Die unterschiedliche Elastizität kann auf verschiedene Weise realisiert werden. Beispielsweise kann sie durch die unterschiedliche Wanddicke der Abschnitte oder unterschiedliche Materialien realisiert werden. Auch können die Sektoren unterschiedlich breit ausgebildet sein, was durch eine Ausweitung der Beabstandung (der Spalte im Bereich der Abschnitte erzielt werden kann). Anders ausgedrückt, sind die Abschnitte eingeschnürt oder tailliert, dort wo sie elastischer sein sollen.

Bei einer Ausführungsform kann die Klemmhülse so gestaltet sein, dass, falls sie beispielsweise in einem Bohrfutter eingeführt ist und über das Bohrfutter eine nach innen wirkende Kraft auf sie ausgeübt werden soll, das Bohrfutter nur einen Teil oder einen Bereich der Klemmhülse berührt. Insbesondere kann die Ausführungsform so gestaltet sein, dass das Bohrfutter nur einen Bereich der Klemmhülse berührt, der einen der beiden Längsenden (erstes oder zweites Ende) der Klemmhülse näher ist als dem anderen Längsende. Insbesondere ist der Bereich in der Nähe des ersten (also insbesondere aufgeweiteten) Endes der Klemmhülse. Insbesondere ist der Bohrerkontakt-Abschnitt vorstehend gegenüber zumindest einem der folgenden Abschnitte ausgebildet: erster Verbindungsabschnitt, zweiter Verbindungsabschnitt und Drahtkontakt-Abschnitt. Auf diese Art und Weise wird der Bohrerkontakt-Abschnitt durch Krafteinwirkung in Richtung auf die Längsachse gedrückt, während insbesondere andere Abschnitte nicht mit dem Bohrfutter in Kontakt sind. Insbesondere führt der Bohrerkontakt-Abschnitt eine Art Schwenkbewegung um einen elastischen Bereich des ersten und/oder zweiten Verbindungsabschnittes durch. Je nach Ausbildung der Elastizität des ersten und/oder zweiten Verbindungsabschnittes kann der Umfang der Schwenkbewegung um den ersten Verbindungsabschnitt anders ausfallen als um den zweiten Verbindungsabschnitt. Dies kann dazu genutzt werden, um in einer gewünschten Abfolge einen Kontakt zwischen Bohrer-Kontaktfläche und Bohrer sowie zwischen Draht-Kontaktfläche und Führungsdraht zu erzielen, während das Bohrfutter immer weiter nach innen drängt sich also mehr und mehr der Längsachse der Klemmhülse nähert.

Wie oben ausgeführt, wird die erfindungsgemäße Klemmhülse vorzugsweise bei einem navigierten System genutzt. Für diesen Fall ist es von Vorteil, wenn die Position der Bohrspitze relativ zum Bohrgerät (Bohrmaschine) bekannt ist. Dies geschieht zum Beispiel über einen Kalibrierungsschritt mittels eines zweiten bekannten navigierten Objekts, das die Lage der Bohrerspitze relativ zu den am Kirschnerdraht befestigten Markereinrichtung aufzeigen kann.

Zur Erleichterung der Benutzung und zur Absicherung der Längenkalibrierung bei navigierter Anwendung kann die Hülse zusätzlich noch einen Anschlag für das Bohrfutter aufweisen, um ein hineinrutschen der Hülse in ein zu großes Bohrfutter zu verhindern. Dieser Anschlag steht (radial) nach außen vor. In Längsrichtung der Klemmhülse betrachtet ist somit dieser Bohrfutteranschlag der Bohrspitze näher als das Bohrfutter.

Um die Längenkalibrierung für die Navigation weiter abzusichern indem Bohrer und insbesondere die Bohrspitze definiert relativ zur Klemmhülse positioniert werden, ist vorzugsweise eine Anlagefläche für das Ende des Bohrers vorgesehen, die hierin auch als "Bohrerende-Anlagefläche" bezeichnet wird. Diese Bohrerende-Anlagefläche ist vorzugsweise Bestandteil, insbesondere einstückiger Bestandteil der Klemmhülse und steht vorzugsweise in das Innere der Klemmhülse in Richtung auf die Längsachse, insbesondere radial einwärts vor. Sie steht insbesondere schräg, quer oder senkrecht zur Längserstreckungsrichtung der Klemmhülse vor. Die Bohrerende-Anlagefläche kann Bestandteil des Drahtkontakt-Abschnittes sein und insbesondere durch denjenigen Teil des Drahtkontakt-Abschnittes gebildet werden, der dem ersten Ende der Klemmhülse am nächsten ist. Insbesondere kann es eine Fläche des Drahtkontakt-Abschnitts sein, die dem ersten Ende der Klemmhülse zugewandt ist.

Die vorliegende Erfindung richtet sich weiter auf ein System, das neben der Klemmhülse vorzugsweise auch den kanülierten Bohrer und/oder den Führungsdraht umfasst. Für dieses System gelten die bereits oben beschriebenen Eigenschaften, insbesondere bei Kraftanwendung oder falls keine Kraft angewendet wird. Insbesondere ist dieses System so ausgelegt, dass der Abschnitt zwischen dem Führungsdraht und der Draht-Kontaktfläche sowie der Abstand zwischen dem Bohrer und der Bohrer-Kontaktfläche so gewählt sind, dass sich bei zunehmender Krafteinwirkung von innen nach außen letztendlich ein kraftschlüssiger Kontakt zwischen der Klemmhülse und sowohl dem Bohrer als auch dem Führungsdraht einstellt. Dies kann insbesondere so bewerkstelligt werden, dass die Abstände zwischen Draht-Kontaktfläche und Führungsdraht anders gewählt werden als zwischen Bohrer-Kontaktfläche und Bohrer. Vorzugsweise ist der Abstand zwischen Bohrer-Kontaktfläche und Bohrer, falls keine Kraft ausgeübt wird, größer als der Abstand zwischen Draht-Kontaktfläche und Führungsdraht.

Vorzugsweise ist der Innendurchmesser im Bereich der Bohrerkontaktfläche im kräftefreien Zustand größer als der Bohrerdurchmesser. Vorzugsweise ist der Innendurchmesser im kräftefreien Zustand bei der Drahtkontaktfläche größer als der Drahtdurchmesser.

Das System kann weiter eine Bohrmaschine, ein Bohrgerät oder ein Bohrfutter umfassen, an dem insbesondere eine Markereinrichtung angebracht ist. Die Markereinrichtung umfasst vorzugsweise mehrere Markerelemente insbesondere in definierter relativer Lage zueinander. Die Markerelemente können aktiv Signale aussenden oder Signale reflektieren. Bei den Signalen handelt es sich insbesondere um Strahlen oder Wellen, beispielsweise Infrarotstrahlen oder Ultraschallwellen. Die Signale können von einer Detektionseinrichtung detektiert werden, um die Lage der Markereinrichtung zu bestimmen.
Wird keine eigene Kalibrierung für die Bohrerspitzenposition verwendet, ergibt sich somit bei bekannten Dimensionen aller Gegenstände allein durch die definierte Lage zwischen Markereinrichtung und Bohrmaschine und zwischen Bohrmaschine und Bohrfutter und zwischen Bohrfutter und Klemmhülse sowie zwischen Klemmhülse und Bohrspitze eine bekannte Lage der Bohrspitze durch Detektion der Markereinrichtung.

Die Erfindung ist weiter vorzugsweise auf ein Navigationssystem gerichtet, das eine Datenverarbeitungsvorrichtung und eine Detektionsvorrichtung enthält. Die Detektionsvorrichtung dient der Detektion der vorgenannten Markerelemente, die beispielsweise am Bohrgeräte und/oder an der Klemmhülse angebracht sein können. Die Detektionssignale werden von der Datenverarbeitungsvorrichtung verarbeitet, um die Lage des Bohrers, insbesondere der Bohrerspitze zu bestimmen. Dabei werden insbesondere die aus der bildgeführten Chirurgie (image guided surgery) oder IGS bekannten Prinzipien eingesetzt.

Bei der folgenden detaillierten Beschreibung werden weitere Merkmale der Erfindung offenbart. Merkmale unterschiedlicher Ausführungsformen können miteinander kombiniert werden.
Figur 1 zeigt die Vorgehensweise beim Platzieren eines Führungsdrahts gemäß dem Stand der Technik;
Figur 2 zeigt eine Vorgehensweise mit einem erfindungsgemäßen System, wobei die Klemmhülse hier nicht gezeigt wird;
Figur 3 zeigt ein erfindungsgemäßes System gemäß einer bevorzugten Ausführungsform, das eine erfindungsgemäße Klemmhülse umfasst;
Figur 4 zeigt einen Querschnitt durch die erfindungsgemäße Klemmhülse;
Figur 5 zeigt eine 3D-Ansicht der erfindungsgemäßen Klemmhülse;
Figur 6 zeigt eine andere Ausführungsform des erfindungsgemäßen Systems, das eine erfindungsgemäße Klemmhülse umfasst;
Figur 7 zeigt eine Außenansicht (3D-Ansicht) der Klemmhülse der Figur 6;
Figur 8 zeigt eine Querschnittsansicht der Klemmhülse der Figur 6.

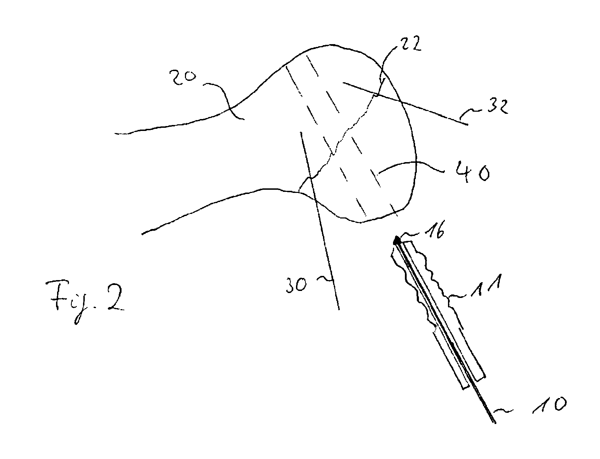

Figur 2 zeigt eine Vorgehensweise mit einem erfindungsgemäßen System, wobei die Klemmhülse hier nicht gezeigt wird. Vorzugsweise wird vorab die Knochenfraktur mit den Kirschnerdrähten 30 und 32 fixiert. Anstelle eines gewöhnlichen Bohrers wird aber ein navigierter Bohrer mit einer Markereinrichtung eingesetzt, so dass die Position der Bohrspitze relativ zum Knochen jederzeit bekannt ist. Es sind also im Gegensatz zum Stand der Technik nicht mehrere Versuche mit einem Kirschnerdraht erforderlich. Durch den kanülierten Bohrer 11 ist der Kirschnerdraht 10 geführt. Eine Spitze 16 des Kirschnerdraht 10 kann von der Spitze des Bohrers 11 vorstehen. Figur 2 zeigt die Situation bevor der Bohrer 11 in den Knochen 20 eingeführt wird, um den Bohrkanal 40 zu erzeugen.

Ausgehend von der in Figur 2 gezeigten Situation wird der kanülierte Bohrer 11 zusammen mit dem Kirschnerdraht 10 gleichzeitig in den Knochen eingebracht. Da der kanülierte Bohrer navigiert wird (z.B. nach den Prinzipien der bildgestützten Navigation in der Chirurgie IGS oder "image guided surgery"), kann die Bohrung 40 in der vorgegebenen Lage erfolgen und insbesondere der Kirschnerdraht kann exakt positioniert werden. Ist der Bohrer mit dem Kirschnerdraht eingebracht, so kann der Kirschnerdraht im Knochen festgeklopft werden, bevor der kanülierte Bohrer 11 abgezogen wird.

Dem Chirurgen stehen üblicherweise kanülierte Bohrer zur Verfügung. Würde er diese beim Bohren der Bohrung navigieren, so könnte er auch ohne Kirschnerdraht die Bohrung an der gewünschten Stelle erzielen. Jedoch setzt sich dann die Kanüle in dem Bohrer mit Knochenmaterial zu, was den Bohrvorgang behindert. Weiter fehlt die führende Unterstützung des Kirschnerdrahts beim Einbringen der Schraube.

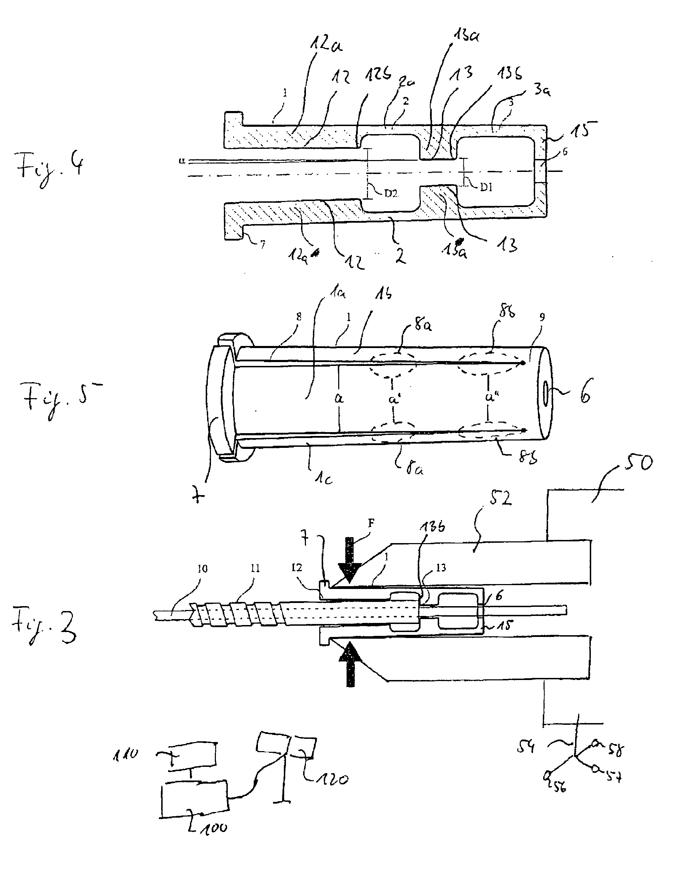

Würde ein Chirurg den Kirschnerdraht lose im kanülierten Bohrer einführen, um obiges zu verhindern, so würde der Kirschnerdraht von dem Knochenmaterial während des Bohrvorgangs von der Bohrspitze weggedrängt werden. Erfindungsgemäß wird deshalb der Kirschnerdraht relativ zum kanülierten Bohrer fixiert. Wie dies gemäß einer erfindungsgemäßen Ausführungsform erfolgt, ist in Figur 3 gezeigt.

Eine erfindungsgemäße Klemmhülse 1 wird z.B. durch eine Kraft F nach innen gedrückt. Die Klemmhülse 1 ist so ausgebildet, dass durch die Kraft F zumindest ein kraftschlüssiger Kontakt zwischen dem Führungsdraht 10 und der Klemmhülse 1 und zwischen dem kanülierten Bohrer 11 und der Klemmhülse 1 erfolgt. Genauer sind zwei Kontaktflächen vorgesehen. Eine Bohrer-Kontaktfläche 12 kontaktiert den Bohrer 11, um eine kraftschlüssige Verbindung zu dem Bohrer 11 zu erzielen. Eine Draht-Kontaktfläche 13 kontaktiert den Führungsdraht 10, um eine kraftschlüssige Verbindung zu dem Führungsdraht 10 zu erzielen. Die Kraft F wird durch das Futter eines Bohrgeräts oder einer Bohrmaschine aufgebracht. Hierzu wird die in Figur 3 gezeigte Anordnung in das Futter 52 der Bohrmaschine 50 bzw. des Bohrgeräts 50 eingeführt. Insbesondere kann der Führungsdraht 10 durch das Bohrgerät 50 hindurch laufen. An der Bohrmaschine 50 kann eine Markereinrichtung 54 mit Markerelementen 56, 57 und 58 angebracht sein. Diese Markereinrichtung kann mit einer Detektionseinrichtung (nicht gezeigt) eines Navigationssystems zum Navigieren von Instrumenten detektiert werden. Durch Detektion der Markereinrichtung kann dann der Bohrer und insbesondere die Bohrspitze navigiert werden. Das Futter 52 liegt an einem flanschartig ausgebildeten Anschlag 7 am ersten Ende der Klemmhülse 1 an. Somit ergibt sich eine bekannte relative Lage zwischen der Klemmhülse 1 und der Markereinrichtung 54. Weiter liegt der Bohrer 11 an der Bohrerende-Anlagefläche 13b an, die sich in Figur 3 links von der Draht-Kontaktfläche 13 befindet. Somit ergibt sich eine bekannte relative Lage zwischen der Klemmhülse 1 und dem Bohrer 11. Da der Abstand zwischen der Bohrspitze an einem Ende des Bohrers und zwischen dem anderen Ende des Bohrers 11 bekannt ist, ist somit die relative Lage zwischen Bohrerspitze und der Markereinrichtung 54 bekannt, so dass die Bohrerspitze durch Detektion der Markereinrichtung 54 navigiert werden kann. Zur Registrierung der Bohrspitze im Bezugssystem des Navigationssystems und insbesondere zur Bestimmung der Lage der Bohrspitze relativ zur Markereinrichtung wird vorzugsweise ein Scanvorgang durchgeführt, bei dem die Lage von Oberflächenpunkten oder Bereichen der Bohrspitze und/oder des Bohrers 11 und/oder der Klemmhülse 1 erfasst werden. Hierzu wird beispielsweise eine Kalibriermatrix eingesetzt, wie sie üblicherweise bei der Kalibrierung / Validierung von Werkzeugen oder Implantaten eingesetzt wird. Diese Kalibriematrix kann insbesondere dazu genutzt werden die Lage der Spitze und die Form des Instruments, hier der Bohrspitze und/oder des Bohrers und/oder der Klemmhülse zu bestimmen. Alternativ zu der oben genannten Vorgehensweise kann beispielsweise die Bohrerspitze durch einen so genannten Pointer oder ein beliebiges anders Werkzeugs bekannter Geometrie von dem Navigationssystem erfasst werden. Der Pointer oder das Werkzeug umfasst mindestens zwei Marker, deren Detektion die Bestimmung der Lage eines speziellen Punktes, z. B. der Pointerspitze erlaubt. Wenn der definierte Punkt an der Bohrspitze anliegt, ist somit die Lage der Bohrerspitze detektiert. Insbesondere ist sie im Bezugssystem des Navigationssystems registriert. Insbesondere ist somit die Lage der Bohrspitze relativ zur Markereinrichtung 54 bekannt.

Wie in Figur 3 gezeigt ist, kann die Markereinrichtung 54 mittels einer Detektionseinrichtung 120 detektiert werden. Die Detektionseinrichtung 120 ist Bestandteil eines Navigationssystems, das insbesondere eine Datenverarbeitungseinrichtung 100 umfasst, die die detektierten Signale verarbeitet, um den Ort der Markeranordnungen bzw. Markereinrichtungen zu bestimmen. Insbesondere kann das Navigationssystem den Ort der Bohrerspitze bestimmen. Der Monitor 110 kann zur Anzeige des Orts der Bohrerspitze, beispielsweise relativ zu einem Knochen 20, an dem beispielsweise auch eine Markereinrichtung angeordnet sein kann, verwendet werden.

Figur 4 zeigt eine Klemmhülse 1 im Querschnitt. Die Längsachse der Klemmhülse ist mit einer Strichpunktlinie gezeigt. Die Klemmhülse weitet sich nach links auf und zwar in einem Winkel α relativ zur Längsachse. Die Kontaktflächen 12 und 13 sind über einen Steg 2 verbunden. Die Draht-Kontaktfläche 13 ist mit dem rechten Ende 15 über einen Steg 3 verbunden. Das rechte Ende 15 ist dem Bohrgerät 50 zugewandt. Die Stege bzw. Verbindungsabschnitte 2 und 3 können unterschiedlich lang und/oder unterschiedlich dick und/oder aus einem unterschiedlichen Material ausgebildet sein. Auf diese Art und Weise lässt sich eine Starrheit oder Elastizität zwischen dem Ende 15 und der Draht-Kontaktfläche 13 einstellen, die sich von der Starrheit oder Elastizität unterscheidet, die zwischen der Draht-Kontaktfläche 13 und der Bohrer-Kontaktfläche 12 gegeben ist. Durch Einstellen einer unterschiedlichen Elastizität und/oder durch Einstellen des Öffnungswinkels α und/oder durch die Größe der lichten Weiten D1 und D2 kann eingestellt werden, bei welchem äußeren Kraftaufwand F sich der Kontakt zwischen der Draht-Kontaktfläche 13 und dem Führungsdraht 10 und zwischen der Bohrer-Kontaktfläche 12 und dem kanülierten Bohrer 11 einstellt und/oder wie weit die Klemmhülse zusammengedrückt werden muss, um den genannten Kontakt zu gewährleisten.

Insbesondere sind die Verbindungsabschnitte bzw. Stege 2 und 3 elastischer ausgebildet als die Bereiche 12a und 13a, die radial auswärts von den Kontaktflächen 12 und 13 jeweils liegen.

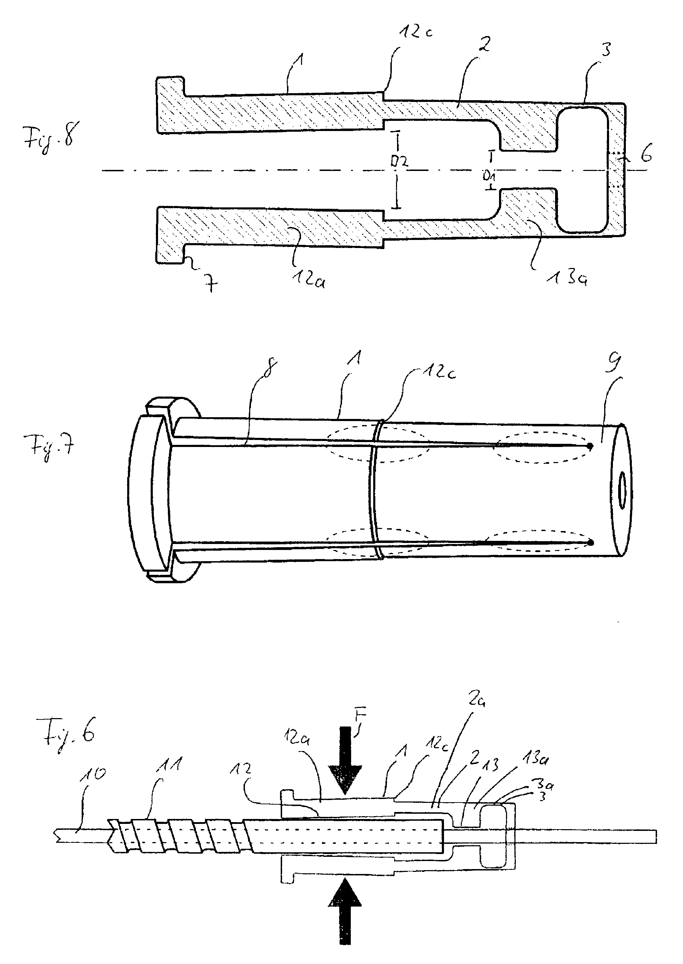

Wird nun eine Kraft F, wie in Figur 3 gezeigt, über ein Bohrfutter auf die Klemmhülse 1 ausgeübt, so schwenkt das der Bohrerseite zugewandte (linke, siehe Figur 3) Ende der Klemmhülse nach innen, so dass sich ein Winkel β (nicht gezeigt) zwischen Bohrer-Kontaktfläche und Längsachse der Klemmhülse in Längserstreckungsrichtung der Klemmhülse verkleinert. In der in Figur 4 gezeigten Situation, ohne äußere Krafteinwirkung ist der Winkel β gleich dem gezeigten Winkel α. Die Klemmhülse ist also vorzugsweise elastisch ausgebildet und erlaubt ein Schwenken der Hülse um das dem Bohrer abgewandte (rechte, siehe Figur 3) Ende 15, so dass sich der Winkel β verringert. Der Winkel α ist der Winkel zwischen der Draht-Kontaktfläche und der Längsachse, gesehen ins Längserstreckungsrichtung der Klemmhülse. Dieser Winkel α verringert sich mit zunehmender Kraft F zunächst weniger als der Winkel β, wie in Figur 6 zu sehen ist. Dies liegt daran, dass auch durch die Kraft F eine Schwenkbewegung um etwa die Mitte des Steges 2 bewirkt wird.

Wird nun Kraft auf die Klemmhülse 1 aufgebracht, so ergibt sich die oben beschriebene Schwenkbewegung, bis ein rechtes Ende 12b (also ein dem Bohrer zugewandtes Ende) der Bohrerkontaktfläche mit dem Bohrer in Kontakt kommt und/oder bis ein rechtes Ende 13b der Draht-Kontaktfläche mit dem Führungsdraht in Kontakt kommt. Da die Verbindungsabschnitte 2 und 3 elastischer ausgebildet sind als die Abschnitte 12a und 13a, die jeweils der Bohrer-Kontaktfläche oder der Draht-Kontaktfläche zugeordnet sind, kann bei sich steigernder Kraft F ein Knick bzw. Schwenkvorgang um ca. die Mitte 2a und 3a der Verbindungsabschnitte 2 und 3 erfolgen, bis die Bohrer-Kontaktfläche 12 satt an dem Bohrer 11 anliegt und die Draht-Kontaktfläche 13 satt an dem Führungsdraht 10 anliegt.

Figur 5 zeigt eine Außenansicht der erfindungsgemäßen Klemmhülse. Die Klemmhülse ist in Längsrichtung vorzugsweise in verschiedene Sektoren unterteilt. Die Sektoren sind durch in Längsrichtung spitz zulaufende Schlitze 8 getrennt. Die Schlitze 8 laufen nach rechts also in Richtung auf den Bohrer zu. Am rechten Ende 9 sind die einzelnen Sektoren miteinander verbunden. Werden die Sektoren durch eine äußere Krafteinwirkung, beispielsweise eines Bohrfutters so zusammengepresst, dass sich die Schlitze schließen, so kann die Außenform der Klemmhülse vorzugsweise zylindrisch werden. Vorzugsweise bilden die Draht-Kontaktflächen und/oder die Bohrer-Kontaktflächen insbesondere entlang des Umfangs geschlossene Zylinderflächen aus, und zwar im zusammengepressten Zustand.

Am linken vom Bohrer abgewandten Ende der Klemmhülse befindet sich ein Vorsprung, der radial nach außen vorsteht und insbesondere als Anschlagfläche 7 für das Bohrfutter 52 dient, genauer als Anschlagsfläche 7 für dasjenige Ende des Bohrfutters 52 dient, das dem Bohrgerät abgewandt ist. Dies ist in Figur 3 zu sehen. Der radial nach außen stehende Fortsatz, der die Anschlagfläche 7 bildet, ist beispielsweise flanschähnlich ausgebildet. An dem anderen Ende der Klemmhülse befindet sich eine Öffnung 6, die vorgesehen ist, um den Führungsdraht 10 hindurchzuführen, wie aus Figur 3 ersichtlich ist. In Figur 5 ist gestrichelt eine alternative Ausführungsform der Schlitze gezeichnet. Die Schlitze 8 laufen demgemäß nicht durchgängig von links nach rechts spitz zu sondern die Schlitze umfassen Ausbuchtungen 8a und 8b. Diese Ausbuchtungen 8a und 8b führen dazu, dass ein Sektor 1 a der Klemmhülse in dem Bereich der Verbindungsabschnitte 2 und 3 in Umfangsrichtung der Klemmhülse schmäler wird. Während der Sektor 1a außerhalb der Verbindungsabschnitte 2 und 3 eine Breite a in Umfangsrichtung hat, ist diese Breite im Bereich der Verbindungsabschnitte 2 und 3 aufgrund der Ausnehmungen 8a und 8b auf die Breite a' und a" verringert. Dies führt zu einer erhöhten Elastizität des Sektorabschnitts im Bereich der Verbindungsabschnitte 2 und 3.

Figur 6 zeigt eine weitere Ausführungsform. Die Verbindungsabschnitte 2 und 3 sind in Figur 6 anders ausgebildet als in Figur 3. In Figur 3 sind sie gleich dick eingezeichnet. In Figur 6 ist der Verbindungsabschnitt 2 dicker als der Verbindungsabschnitt 3. Das führt dazu, dass der Verbindungsabschnitt 2 steifer ist als der Verbindungsabschnitt 3. Bei Einwirkung einer äußeren Kraft F kommt es deshalb zuerst und/oder verstärkt in einem mittleren Bereich 3a des Verbindungsabschnitts 3 zu einem Abknicken. Der mittlere Bereich 2a der Verbindungsabschnitts 2 knickt weniger und/oder erst bei einer weiteren Erhöhung der Kraft ab. Ist der Abstand zwischen Bohrer-Kontaktfläche 12 und Bohrer 11 sowie zwischen Draht-Kontaktfläche 13 und Führungsdraht 10 passend gewählt, so ergibt sich beim satten Anliegen der Bohrer-Kontaktfläche 12 an den Bohrer 11 ebenfalls ein sattes Anliegen der Draht-Kontaktfläche 13 an dem Führungsdraht 10. Aufgrund der höheren Elastizität des Bereichs 3a im Vergleich zum Bereich 2a und da der Abschnitt 12a, der der Bohrer-Kontaktfläche 12 zugeordnet ist, radial gegenüber dem Abschnitt 13a, der der Draht-Kontaktfläche zugeordnet ist, (und insbesondere auch gegenüber den Verbindungsabschnitten 2 und 3) nach außen vorsteht, wirkt auf diesen Abschnitt 12a zuerst die Kraft F. Dies führt zu einem Schwenk- oder Knickvorgang um den Bereich 3a, der verformbarer oder elastischer ausgebildet ist, als der Abschnitt 2a. Somit ergibt sich zuerst ein Kontakt des linksseitigen Endes des Abschnitts 12a mit dem kanülierten Bohrer 11. Bei weiterer Krafteinwirkung wird die Kontaktfläche 12 immer mehr parallel zu der Längserstreckungsrichtung des Bohrers 11. Dies führt gleichzeitig dazu, dass die Draht-Kontaktfläche immer weiter abgesenkt wird, bis sie schließlich ebenfalls den Führungsdraht 10 kontaktiert, so dass sich eine kraftschlüssige Verbindung zwischen der Klemmhülse und dem Führungsdraht ergibt. Bei weiterem absenken bis zu Kraft- und Formschluss zwischen der Kontaktfläche 12 und dem kanülierten Bohrer 11, ergibt sich an der kraft- und formschlüssigen Verbindung zwischen der Klemmbacke 13 und dem Draht die maximal erreichbare Klemmkraft.

Der Bohrer-Kontaktfläche zugehörige Abschnitt 12a ist um eine Kante 12c nach außen vorstehend. Diese Kante 12c ist auch gut in der Außenansicht der Klemmhülse 1 in Figur 7 zu erkennen. Im Übrigen ist die Klemmhülse 1 so aufgebaut, wie in Figur 5 zu sehen ist.

Figur 8 zeigt nochmals vergrößert den Querschnitt der Klemmhülse, wie sie in Figur 6 gezeigt ist. Die lichten Weiten D2 und D1, die jeweils durch die Bohrer-Kontaktfläche 12 und die Draht-Kontaktfläche 13 definiert sind, werden passend gewählt, um bei Krafteinwirkung einen satten Kontakt mit dem Bohrer bzw. dem Führungsdraht zu erzielen.

## Patentansprüche

1. Klemmhülse (1) zum Klemmen eines kanülierten Bohrers (11) und eines Führungsdrahtes (10), insbesondere Kirschnerdrahtes, für medizinische Zwecke, wobei die Klemmhülse (1) in das Bohrfutter einer Bohrmaschine einbringbar ist;
mit mindestens einer Bohrer-Kontaktfläche (12), die in das Innere der Klemmhülse vorsteht, um bei einer auf die Außenseite der Klemmhülse nach innen wirkenden Kraft den kanülierten Bohrer (11), durch den der Führungsdraht (10) verläuft, zu klemmen;
mit mindestens einer Draht-Kontaktfläche (13), um bei der auf die Außenseite der Klemmhülse (1) nach innen wirkenden Kraft einen freiliegenden, nicht vom kanülierten Bohrer (11) umhüllten Teil des Führungsdraht zu klemmen;
wobei die mindestens eine Draht-Kontaktfläche (13) und die mindestens eine Bohrer-Kontaktfläche (12) in Längsrichtung der Klemmhülse (1) hintereinander liegen.

2. Klemmhülse nach Anspruch 1, bei welcher die mindestens eine Draht-Kontaktfläche (13) weiter nach innen vorsteht als die mindestens eine Bohrer-Kontaktfläche (12).

3. Klemmhülse nach einem der vorhergehenden Ansprüche, bei welchem die mindestens eine Draht-Kontaktfläche (13) und die mindestens eine Bohrer-Kontaktfläche (12) als Zylinderflächenabschnitt mit Zylinderradius ausgebildet ist, wobei der Zylinderradius der mindestens einen Draht-Kontaktfläche (13) kleiner ist als der Zylinderradius der mindestens einen Bohrer-Kontaktfläche (12).

4. Klemmhülse nach einem der vorhergehenden Ansprüche, bei welchem die mindestens eine Draht-Kontaktfläche (13) und/oder die mindestens eine Bohrer-Kontaktfläche (12) so ausgebildet sind, dass sich deren Oberflächenpunkte in Längsrichtung der Klemmhülse in Richtung eines ersten Längsendes der Klemmhülse zumindest abschnittsweise stetig oder stufenartig zunehmend von der Längsache der Klemmhülse (1) entfernen, falls keine Kraft (F) nach Innen wirkt.

5. Klemmhülse nach einem der vorhergehenden Ansprüche, bei welchem die Klemmhülse in Längsrichtung verlaufende Sektoren (1a, 1 b, 1c) aufweist, die die mindestens eine Bohrer-Kontaktfläche (12) und die mindestens eine Draht-Kontaktfläche (13) umfassen und von dem ersten Längsende der Klemmhülse bis zu einem Endabschnitt der Klemmhülse, der an einem zweiten Längsende der Klemmhülse liegt, verlaufen, wobei die Sektoren (1a, 1 b, 1 c) bei keiner nach innen wirkenden Kraft voneinander beabstandet sind.

6. Klemmhülse nach Anspruch 5, bei welchem die mindestens eine Draht-Kontaktfläche (13) und/oder die mindestens eine Bohrer-Kontaktfläche (12) so ausgebildet sind, dass deren Oberflächenpunkte in Längsrichtung der Klemmhülse (1) in Richtung des ersten Längsendes parallel zur Längsache der Klemmhülse (1) verlaufen, falls sich die Sektoren (1a, 1b, 1c) auf Grund einer nach Innen wirkenden Kraft (F) näher kommen oder kontaktieren.

7. Klemmhülse nach einem der vorhergehenden Abschnitte, bei welchem die Sektoren elastisch ausgebildet sind, so dass durch eine radial nach innen wirkender Kraft (F) die Sektoren (1a, 1b, 1c) zumindest teilweise durch Überwindung der Beabstandung in Kontakt bringbar sind.

8. Klemmhülse nach einem der vorhergehenden Ansprüche, bei welcher ein Drahtkontakt-Abschnitt (13a) eine der mindestens einen Draht-Kontaktflächen (13) umfasst und über einen ersten Verbindungsabschnitt (3) mit dem zweiten Längsende der Klemmhülse (1) verbunden ist und mit einem zweiten Verbindungsabschnitt (2) mit einem Bohrerkontakt-Abschnitt (12a), der eine der mindestens einen Bohrer-Kontaktflächen (12) umfasst, verbunden ist, wobei zumindest ein Teil des ersten und/oder zweiten Verbindungsabschnittes (2, 3) elastischer ausgebildet ist als der Bohrerkontakt-Abschnitt und/oder als der Drahtkontakt-Abschnitt.

9. Klemmhülse nach einem der vorhergehenden Ansprüche, bei welcher der Bohrerkontakt-Abschnitt (12a) der Klemmhülse, der die Bohrer-Kontaktfläche (12) aber nicht die Draht-Kontaktfläche (13) umfasst, bei keinerlei äußerer Kraft (F) auf die Klemmhülse einen anderen Außendurchmesser aufweist, als ein Drahtkontakt-Abschnitt (13a), der die Draht-Kontaktfläche (13) aber nicht die Bohrer-Kontaktfläche (12) umfasst, und/oder bei welcher der Außendurchmesser des Bohrerkontakt-Abschnittes (12a) größer ist, als der des Drahtkontakt-Abschnittes (13a) und/oder außen liegende Oberflächenpunkte der Klemmhülse sich in Richtung eines ersten Längsendes der Klemmhülse sich zumindest abschnittsweise stetig oder stufenartig zunehmend von der Längsachse der Klemmhülse entfernen.

10. Klemmhülse nach einem der vorhergehenden Ansprüche, bei welchem der Drahtkontakt-Abschnitt (13a) eine Bohrerende-Anlagefläche (13b) aufweist, die in Längsrichtung der Klemmhülse zwischen einer der mindestens einen Bohrer-Kontaktflächen (12) und einer der mindestens einen Draht-Kontaktflächen (13) liegt und senkrecht oder quer zur Längsrichtung der Klemmhülse (1) nach innen vorsteht, aber nicht weiter nach innen vorsteht als die Draht-Kontaktfläche (13).

11. Klemmhülse nach einem der vorhergehenden Ansprüche, mit einem Bohrfutteranschlag (7), der radial am ersten Längsende der Klemmhülse nach Außen vorsteht.

12. System aus kanülierten Bohrer (11), Führungsdraht (10) und der Klemmhülse (1) nach einem der vorhergehenden Ansprüche, bei welchem, falls die Klemmhülse (1) den kanülierten Bohrer (11) mit innenliegendem Führungsdraht (10) umgibt, um beide, falls eine Kraft (F) nach Innen wirkt, zu fixieren, wobei, falls keine Kraft nach Innen wirkt , der Abstand der Draht-Kontaktfläche (13) zum Führungsdraht (10) anders, also größer oder kleiner ist als Abstand der Bohrer-Kontaktfläche (12) zum kanülierten Bohrer (11) und/oder die lichte Weite im Bereich der Bohrer-Kontaktflächen größer ist als der Durchmesser des Bohrers (11), falls keine Kraft nach Innen wirkt, und/oder die lichte Weite im Bereich der Draht-Kontaktfläche größer ist als der Durchmesser des Führungsdrahtes (10), falls keine Kraft nach Innen wirkt.

13. System nach Anspruch 12,
das weiter ein Bohrgerät (50) mit Bohrfutter (52) umfasst, wobei die Klemmhülse (1) in das Bohrfutter (52) einbringbar ist und wobei an dem Bohrgerät (50) eine Markereinrichtung (54) angebracht ist.

14. Navigationssystem mit einer Datenverarbeitungsvorrichtung (100), mit einer Detektionsvorrichtung (120) zum Detektieren von Markern (54, 60) und mit der Klemmhülse nach einem der Ansprüche 1 bis 11 oder dem System nach einem der Ansprüche 12 oder 13.

## Claims

1. A clamping sleeve (1) for clamping a cannulated drill (11) and a guide wire (10), in particular a Kirschner wire, for medical purposes, wherein the clamping sleeve (1) can be introduced into the chuck of a drilling machine, comprising:
at least one drill contact area (12) which protrudes into the interior of the clamping sleeve in order to clamp the cannulated drill (11), through which the guide wire (10) runs, when a force acts inwards on the outer side of the clamping sleeve;
at least one wire contact area ( 13) in order to clamp an exposed part of the guide wire which is not enveloped by the cannulated drill (11) when the force acts inwards on the outer side of the clamping sleeve (1);
wherein the at least one wire contact area (13) and the at least one drill contact area (12) lie sequentially in the longitudinal direction of the clamping sleeve (1).

2. The clamping sleeve according to claim 1, wherein the at least one wire contact area (13) protrudes further inwards than the at least one drill contact area (12).

3. The clamping sleeve according to any one of the preceding claims, wherein the at least one wire contact area (13) and the at least one drill contact area (12) are formed as a cylindrical area portion having a cylindrical radius, wherein the cylindrical radius of the at least one wire contact area (13) is smaller than the cylindrical radius of the at least one drill contact area (12).

4. The clamping sleeve according to any one of the preceding claims, wherein the at least one wire contact area (13) and/or the at least one drill contact area (12) are formed such that their surface points are increasingly distant, continuously or incrementally at least in portions, from the longitudinal axis of the clamping sleeve (1) in the longitudinal direction of the clamping sleeve in the direction of a first longitudinal end of the clamping sleeve, if there is no force (F) acting inwards.

5. The clamping sleeve according to any one of the preceding claims, wherein the clamping sleeve comprises sectors (1a, 1b, 1c) running in the longitudinal direction, which comprise the at least one drill contact area (12) and the at least one wire contact area (13) and run from the first longitudinal end of the clamping sleeve to an end portion of the clamping sleeve which lies at a second longitudinal end of the clamping sleeve, wherein the sectors (1a, 1b, 1c) are spaced apart from each other when there is no force acting inwards.

6. The clamping sleeve according to claim 5, wherein the at least one wire contact area (13) and/or the at least one drill contact area (12) are formed such that their surface points run parallel to the longitudinal axis of the clamping sleeve (1) in the longitudinal direction of the clamping sleeve (1) in the direction of the first longitudinal end, if the sectors (1a, 1b, 1c) approach or contact each other due to a force (F) acting inwards.

7. The clamping sleeve according to any one of the preceding claims, wherein the sectors are formed to be elastic, such that a force (F) acting radially inwards can move the sectors (1a, 1b, 1c) into contact, at least partially by overcoming the spacing.

8. The clamping sleeve according to any one of the preceding claims, wherein a wire contact portion (13a) comprises one of the at least one wire contact areas (13) and is connected to the second longitudinal end of the clamping sleeve (1) via a first connecting portion (3) and to a drill contact portion (12a) by a second connecting portion (2), wherein the drill contact portion (12a) comprises one of the at least one drill contact areas (12) and at least a part of the first and/or second connecting portion (2, 3) is formed to be more elastic than the drill contact portion and/or the wire contact portion.

9. The clamping sleeve according to any one of the preceding claims, wherein the drill contact portion (12a) of the clamping sleeve, which comprises the drill contact area (12) but not the wire contact area (13), exhibits a different outer diameter to a wire contact portion (13a), which comprises the wire contact area (13) but not the drill contact area (12), when there is not any external force (F) on the clamping sleeve, and/or wherein the outer diameter of the drill contact portion (12a) is greater than that of the wire contact portion (13a) and/or exterior surface points of the clamping sleeve are increasingly distant, continuously or incrementally at least in portions, from the longitudinal axis of the clamping sleeve in the direction of a first longitudinal end of the clamping sleeve.

10. The clamping sleeve according to any one of the preceding claims, wherein the wire contact portion (13a) comprises a drill end abutting area (13b) which lies between one of the at least one drill contact areas (12) and one of the at least one wire contact areas (13) in the longitudinal direction of the clamping sleeve and protrudes inwards, perpendicular or transverse to the longitudinal direction of the clamping sleeve (1), but does not protrude further inwards than the wire contact area (13).

11. The clamping sleeve according to any one of the preceding claims, comprising a chuck stopper (7) which protrudes radially outwards at the first longitudinal end of the clamping sleeve.

12. A system consisting of the cannulated drill (11), the guide wire (10) and the clamping sleeve (1) according to any one of the preceding claims, wherein if the clamping sleeve (1) surrounds the cannulated drill (11) comprising an interior guide wire (10), both are fixed if a force (F) acts inwards, wherein if there is no force acting inwards, the distance between the wire contact area (13) and the guide wire (10) is different to, i.e. greater or smaller than, the distance between the drill contact area (12) and the cannulated drill (11), and/or the clear width in the region of the drill contact areas (12) is greater than the diameter of the drill (11) if there is no force acting inwards, and/or the clear width in the region of the wire contact area is greater than the diameter of the guide wire (10) if there is no force acting inwards.

13. The system according to claim 12, further comprising a drilling apparatus (50) including a chuck (52), wherein the clamping sleeve (1) can be introduced into the chuck (52) and wherein a marker device (54) is attached to the drilling apparatus (50).

14. A navigation system, comprising: a data processing device (100) and a detection device (120) for detecting markers (54, 60); and the clamping sleeve according to any one of claims 1 to 11 or the system according to claims 12 or 13.

## Revendications

1. Manchon de serrage (1) pour serrer une mèche tubulaire (11) et une broche de guidage (10), en particulier une broche de Kirschner, à des fins médicales, dans lequel le manchon de serrage (1) peut être introduit dans le mandrin de mèche d'une perceuse,
comportant au moins une aire de contact de mèche (12) qui fait saillie dans l'intérieur du manchon de serrage afin de serrer la mèche tubulaire (11), à travers laquelle la broche de guidage s'étend, lorsqu'une force agit vers l'intérieur sur le côté extérieur du manchon de serrage,
comportant au moins une aire de contact de broche (13) afin de serrer une partie exposée de la broche de guidage qui n'est pas enveloppée par la mèche tubulaire (11) lorsque la force agit vers l'intérieur sur le côté extérieur du manchon de serrage (1),
dans lequel la au moins une aire de contact de broche (13) et la au moins une aire de contact de mèche (12) sont l'une derrière l'autre dans la direction longitudinale du manchon de serrage (1).

2. Manchon de serrage selon la revendication 1, dans lequel la au moins une aire de contact de broche (13) fait saillie vers l'intérieur plus loin que la au moins une aire de contact de mèche (12).

3. Manchon de serrage selon l'une quelconque des revendications précédentes, dans lequel la au moins une aire de contact de broche (13) et la au moins une aire de contact de mèche (12) sont configurées sous la forme d'une portion de zone cylindrique ayant un rayon de cylindre, dans lequel le rayon de cylindre de la au moins une aire de contact de broche (13) est plus petit que le rayon de cylindre de la au moins une aire de contact de mèche (12).

4. Manchon de serrage selon l'une quelconque des revendications précédentes, dans lequel la au moins une aire de contact de broche (13) et/ou la au moins une aire de contact de mèche (12) sont configurées de telle sorte que leurs points de surface sont de plus en plus éloignés, de manière continue ou incrémentielle au moins par portions, depuis l'axe longitudinal du manchon de serrage (1) dans la direction longitudinale du manchon de serrage en direction d'une première extrémité longitudinale du manchon de serrage, si aucune force (F) n'agit vers l'intérieur.

5. Manchon de serrage selon l'une quelconque des revendications précédentes, dans lequel le manchon de serrage comporte des secteurs (1a, 1b, 1c) s'étendant dans la direction longitudinale, lesquels secteurs comportent la au moins une aire de contact de mèche (12) et la au moins une aire de contact de broche (13) et s'étendent à partir de la première extrémité longitudinale du manchon de serrage jusqu'à une portion d'extrémité du manchon de serrage qui se situe sur une seconde extrémité longitudinale du manchon de serrage, dans lequel les secteurs (1a, 1b, 1c) sont espacés les uns des autres lorsque aucune force n'agit vers l'intérieur.

6. Manchon de serrage selon la revendication 5, dans lequel la au moins une aire de contact de broche (13) et/ou la au moins une aire de contact de mèche (12) sont formées de telle sorte que leurs points de surface s'étendent parallèlement à l'axe longitudinal du manchon de serrage (1) dans la direction longitudinale du manchon de serrage (1) en direction de la première extrémité longitudinale, si les secteurs (1a, 1b, 1c) se rapprochent les uns des autres ou entrent en contact les uns avec les autres en raison d'une force (F) agissant vers l'intérieur.

7. Manchon de serrage selon l'une quelconque des revendications précédentes, dans lequel les secteurs sont formés de manière élastique, de telle sorte que les secteurs (1a, 1b, 1c) peuvent être mis en contact, au moins partiellement, en surmontant l'espacement au moyen d'une force (F) agissant radialement vers l'intérieur.

8. Manchon de serrage selon l'une quelconque des revendications précédentes, dans lequel une portion de contact de broche (13a) comporte l'une des au moins une aire de contact de broche (13) et est reliée à la seconde extrémité longitudinale du manchon de serrage (1) par une première portion de liaison (3), et est reliée à une portion de contact de mèche (12a), laquelle comporte l'une des au moins une aire de contact de mèche (12), par une seconde portion de liaison (2), dans lequel au moins une partie de la première et/ou seconde portion de liaison (2, 3) est formée de manière plus élastique que la portion de contact de mèche et/ou la portion de contact de broche.

9. Manchon de serrage selon l'une quelconque des revendications précédentes, dans lequel la portion de contact de mèche (12a) du manchon de serrage, laquelle comporte l'aire de contact de mèche (12) mais pas l'aire de contact de broche (13), présente un diamètre extérieur différent d'une portion de contact de broche (13a), laquelle comporte l'aire de contact de broche (13) mais pas l'aire de contact de mèche (12), lorsque aucune force extérieure (F) n'est appliquée sur le manchon de serrage, et/ou dans lequel le diamètre extérieur de la portion de contact de mèche (12a) est plus grand que celui de la portion de contact de broche (13a), et/ou des points de surface extérieurs du manchon de serrage sont de plus en plus éloignés, de manière continue ou incrémentielle au moins par portions, depuis l'axe longitudinal du manchon de serrage en direction d'une première extrémité longitudinale du manchon de serrage.

10. Manchon de serrage selon l'une quelconque des revendications précédentes, dans lequel la portion de contact de broche (13a) comporte une surface de butée d'extrémité de mèche (13b) qui se situe entre l'une des au moins une aire de contact de mèche (12) et l'une des au moins une aire de contact de broche (13) dans la direction longitudinale du manchon de serrage, et fait saillie vers l'intérieur, perpendiculairement ou transversalement à la direction longitudinale du manchon de serrage (1), mais ne fait pas saillie plus loin vers l'intérieur que l'aire de contact de broche (13).

11. Manchon de serrage selon l'une quelconque des revendications précédentes, comportant une butée de mandrin de mèche (7) qui fait radialement saillie vers l'extérieur sur la première extrémité longitudinale du manchon de serrage.

12. Système constitué de la mèche tubulaire (11), de la broche de guidage (10) et du manchon de serrage (1) selon l'une quelconque des revendications précédentes, dans lequel, si le manchon de serrage (1) entoure la mèche tubulaire (11) avec la broche de guidage (10) à l'intérieur, les deux sont fixés si une force (F) agit vers l'intérieur, dans lequel, si aucune force n'agit vers l'intérieur, la distance entre l'aire de contact de broche (13) et la broche de guidage (10) est différente, c'est-à-dire qu'elle est plus grande ou plus petite, de la distance entre l'aire de contact de mèche (12) et la mèche tubulaire (11), et/ou la largeur d'ouverture dans la zone de l'aire de contact de mèche est plus grande que le diamètre de la mèche (11) si aucune force n'agit vers l'intérieur, et/ou la largeur d'ouverture dans la zone de l'aire de contact de broche est plus grande que le diamètre de la broche de guidage (10) si aucune force n'agit vers l'intérieur.

13. Système selon la revendication 12, comportant également un appareil de perçage (50) muni d'un mandrin de mèche (52), dans lequel le manchon de serrage (1) peut être introduit dans le mandrin de mèche (52), et dans lequel un dispositif de marquage (54) peut être fixé sur l'appareil de perçage (50).

14. Système de navigation comportant un dispositif de traitement de données (100), un dispositif de détection (120) pour détecter des marqueurs (54, 60) et le manchon de serrage selon l'une quelconque des revendications 1 à 11 ou le système selon l'une quelconque des revendications 12 ou 13.
